# FASCICULE DE BREVET EUROPEEN

(11) **EP 0 278 821 B1**
(45) Date de publication et mention de la délivrance du brevet: **05.10.1994**
(21) Numéro de dépôt: 88400132.2
(22) Date de dépôt: 21.01.1988
(51) Int. Cl.: A61K 31/485, A61K 37/02, A61K 37/24, A61K 33/30

(54) **Utilisation d'antagonistes de la morphine dans la préparation de médicaments à effet immunomodulateur et anti-viral, destinés notamment à traiter les états immuno-déficitaires acquis**
Verwendung von Morphinantagonisten zur Herstellung von Medikamenten mit immunmodulatorischer und antiviraler Wirkung, insbesondere bestimmt zur Behandlung von erworbenen Immunmangelzuständen
Use of morphine antagonists in the preparation of medicaments with immuno-modulating and antiviral effects, especially for treating acquired immuno-deficiency syndroms

(30) Priorité: 21.01.1987 FR 8700670
(43) Date de publication de la demande: 17.08.1988
(73) Titulaire: Shelly, Marc Yves Franck Clément, 75006 Paris (FR)
(72) Inventeur: Shelly, Marc Yves Franck Clément, 75006 Paris (FR)
(74) Mandataire: Tonnellier, Jean-Claude

(56) Documents cités:
- PHARMACOLOGY, vol. 14, 1976; W.F.GEBER et al., pp. 322-329*
- ADV. ALCOHOL SUBST. ABUSE (USA), vol. 1, nos. 3-4, 1982, The Haworth Press Inc. (US); A.FALEK et al., pp. 5-20*
- OTOLRYNGOL HEAD NECK SURG., vol. 94, 1986; G.T.WOLF et al., pp. 224-229*
- TRENDS NEUROSCI., vol. 7/7, 1984; K.J.CHANG, pp. 234-235*
- LIFE SCIENCES, vol. 35, 1984, Pergamon Press (US); N.KAY et al., pp. 53-59*
- JOURNAL OF IMMUNOLOGY, vol. 136, no. 3, 01 février 1986, (US); T.N.MANDLER et al., pp. 934-939*
- PSYCHOTHER. PSYCHOSOM., vol. 42, 1984, S. Karger AG, Basel (CH); E.G.FISCHER et al., pp. 195-204*
- CANCER RESEARCH, vol. 41, avril 1981; F.LEGROS et al., pp. 1539-1544*
- JOURNAL OF IMMUNOLOGY, vol. 132, no. 1, janvier 1984, US; H.M.JOHNSON et al., pp. 246-250*
- DIALOG INFORMATION SERVICES, file 155, medline 66-88/May; no. 06408708*

## Description

La présente invention a pour objet l'utilisation des antagonistes de la morphine dans la préparation de médicaments à effet immunomodulateur et anti-viral destinés à traiter les états immunodéficitaires acquis, et en particulier l'infection par le VIH (virus d'immunodéficience humaine).

On sait que les états immuno-déficitaires sont caractérisés notamment par une dépression importante des défenses immunitaires à médiation cellulaire. On a déjà proposé de traiter ces affections à l'aide de nombreux agents ou méthodes thérapeutiques capables de stimuler selon divers mécanismes, l'immunité cellulaire.

L'approche conventionnelle de l'immunostimulation est en fait fondée sur l'hypothèse que les fonctions immunitaires déprimées, dans ces états pathologiques, doivent être stimulées à l'aide de médiateurs généraux de la communication entre cellules immunocompétentes. Ces médiateurs, qui sont dénueés de spécificité physiopathologique étroite, s'avèrent généralement inadaptés dans cette indication, et leurs effets secondaires indésirables conduisent le plus souvent à renoncer à leur utilisation.

Les échecs observés avec les immunostimulants classiques, ne constituent pas une véritable surprise pour les spécialistes, qui connaissent à la fois la grande variété des mécanismes de défense immunitaire, et les interréactions complexes et nombreuses, d'ailleurs loin d'être toutes élucidées, entre ces divers mécanismes. Le concept d'immunostimulant englobe en fait des substances ayant des effets très divers, voire même opposés, sur certaines catégories de cellules immunocompétentes. Pour cette raison, les spécialistes préfèrent souvent appeler ces substances des agents immunomodulateurs.

Les immunologistes savent bien que l'étude in vitro ou même in vivo, mais de toute façon nécessairement fragmentaire, de l'action des agents immunomodulateurs potentiels sur les diverses cellules immunocompétentes, ne permet pas de prévoir quel sera l'effet global de ces agents sur les malades chez lesquels des dysfonctionnements immunitaires sont déjà présents. Et cela est encor plus vrai, bien enterdu, lorsqu' il s'agit de traiter les personnes souffrant de syndromes d'immunodéficience acquise entraînés par l'infection par le VIH, compte tenu des nombreux échecs qui ont déjà été observés dans ce domaine.

D'autre part, on connaît déjà l'utilisation, notamment dans l'infection par le VIH, de substances antivirales telles que des inhibiteurs de la réplication virale, comme l'azidothymidine (AZT). Toutefois, en raison d' effets toxiques importants, les risques liés à ce médicament, en particulier chez les sujets immunodéprimés, en limitent l'utilisation aux cas d'infections graves menaçant le pronostic vital ; voir par exemple D. INGRAND, Médecine et Maladies infectieuses, Novembre 1987, Tome 17, N° 11 Bis, page 672.

Le document ADV. ALCOHOL SUBST. ABUSE (USA), vol. 1, nos. 3-4, 1982 P. 5-20, montre que les opiacés induisent une diminution du nombre de cellules lvmphocytaires capables de former des rosettes avec les globules rouges de mouton. Ce document ne contient aucune indication sur d'éventuelles modifications des fonctions immunitaires de ces cellules.

Le document OTOLARYNGOL. HEAD NECK SURG., Vol. 94, 1986, P. 224-229, concerne l'étude in vitro de l'augmentation de la production des lymphokines par la beta-endorphine chez les patients ayant certains cancers particuliers. Cette étude, qui ne porte pas sur des fragments de beta-endorphine, ne contient qu'une conclusion très générale selon laquelle les peptides neuro-endocriniens pourraient jouer un rôle important dans la régulation du système immunitaire. En outre, l'objet de ce document ne concerne pas la restauration de l'immunité, ni un effet antiviral, dans les états immunodéficitaires acquis.

On a maintenant découvert que, de façon surprenante, les antagonistes de la morphine, doués d'affinité pour les récepteurs opioïdes mu, ou pour les récepteurs mu et kappa, permettent de relever les défenses immunitaires à médiation cellulaire chez les patients souffrant d'états immuno-déficitaires acquis, et en particulier chez les sujets contaminés par le VIH. Ces antagonistes présentent en outre une action antivirale intrinsèque, en particulier contre le VIH.

L'observation fortuite d'une amélioration clinique notable, chez plusieurs sujets atteints d'une infection par le VIH, ex-toxicomanes soumis à un traitement par la Naltrexone, en tant que traitement de soutien après la crise de sevrage, a conduit le demandeur d'une part a' étudier de façon plus approfondie l'action de ce médicament sur le relèvement des défenses immunitaires à médiation cellulaire et son éventuelle action anti-virale, et d'autre part à rechercher si les mêmes effets bénéfiques pouvaient être observés avec d'autres antagonistes de la morphine.

On sait que la morphine et certains opioïdes possèdent une haute affinité de liaison pour les récepteurs mu, et accessoirement les récepteurs kappa, présents sur certaines cellules nerveuses. On sait aussi que des récepteurs mu sont également présents sur certaines cellules immunocompétentes. D'autres substances opioïdes endogènes, notamment la Met-enképhaline, activent des récepteurs dits delta, qui sont également présents à la fois sur des cellules nerveuses et sur certaines cellules immunocompétentes.

Il est actuellement connu que l'activation de récepteurs opioïdes (actuellement appelés récepteurs mu) par la morphine et par certains opiacés exogènes entraîne un effet immunodépresseur, et que l'activation d'autres récepteurs (actuellement appelés récepteurs delta), notamment par la Met-enképhaline, produit un effet immunostimulant ; voir par exemple WYBRAN et al, J. Immunol., 123(3), 1068 (1979).

Bien que le demandeur ne se considère pas comme lié par cette hypothèse, il est possible d'expliquer schématiquement l'effet de stimulation immunitaire et l'action antivirale cliniquement observés avec la Naltrexone, en supposant que des facteurs opiacés (alcaloïdes) et/ou opioïdes (peptides), non identifiés actuellement, exercent un effet immunodépresseur et pro-infectieux médié par l'activation des récepteurs mu, et accessoirement kappa, de type morphinique, et que la Naltrexone antagonise ce double effet.

Afin de vérifier le bien-fondé de cette hypothèse, le demandeur a étudié l'effet du fragment 1-27 de la bêta-endorphine. On sait que la bêta-endorphine, peptide constitué de 31 motifs d'acides aminés, a la propriété d'activer à la fois les récepteurs mu et delta, l'effet morphinomimétique , lié à l'activation du récepteur mu, étant toutefois dominant ; voir par exemple LOH et al., Proc. Natl Acad. Sci. (U.S.A.), 73, 2895 (1976).

On sait aussi que le fragment 1-27 de la bêta-endorphine, qui constitue l'un des produits de dégradation de la bêta-endorphine dans l'organisme, possède un effet antagoniste de celui de la bêta-endorphine vis à vis du récepteur mu ; voir par exemple HAMMONDS et al., Proc. Natl. Acad. Sci. (U.S.A.), 81,1389 (1984).

Or le demandeur a constaté que le fragment 1-27 de la bêta-endorphine permet, tout comme la Naltrexone, de relever les défenses immunitaires à médiation cellulaire tout en exerçant un effet antiviral intrinsèque. Cette observation est donc compatible avec l'hypothèse formulée ci-dessus.

D'autres hypothèses, éventuellement complémentaires, peuvent être faites. Par exemple, l'occupation des sites mu par la Naltrexone ou par le fragment 1-27, peut favoriser l'occupation et l'activation des récepteurs delta par la bêta-endorphine.

Il est également possible que la Naltrexone préserve et même favorise la libération des enképhalines endogènes en se fixant sur les autorécepteurs présynaptiques capables d'inhiber cette libération.

Un effet antiviral intrinsèque a également été observé dans l'infection par le VIH.

En effet, on a observé chez les malades mentionnés précédemment, traités par la Naltrexone, une amélioration notable des symptômes cliniques suivants : fièvre, malaise général et sueurs nocturnes; reprise de l'appétit et du poids. On sait que ces symptômes, dont la disparition ou l'amélioration est ici observée, sont caractéristiques de la réplication virale ; voir par exemple R. YARCHOAN et al, in "AIDS : Modern Concepts and Therapeutic Challenges, S. BRODER Ed., Marcel DEKKER Inc, P 343 (1987).

L'effet antiviral observé dans l'infection par le VIH, avec la Naltrexone, peut être expliqué schématiquement, par exemple, en faisant l'hypothèse que certaines protéines virales se comportent, dans les états immunodéficitaires acquis, comme des substances morphinomimétiques, directement ou par l'intermédiaire de substrats endogènes.

Il convient de souligner que, contrairement aux traitements classiques, le traitement concerné par l'invention par les antagonistes morphiniques revient à exercer d'une part un effet immunomodulateur, non pas en stimulant d'une façon générale les défenses immunitaires, mais en inhibant un effet immunosuppresseur spécifique à la maladie, et d'autre part une activité antivirale. Dans ce cas particulier, la distinction thérapeutique entre ces deux effets immunomodulateur et antiviral n'est plus possible, les antagonistes morphiniques étant dotés de la double compétence pharmacologique.

L'ensemble des observations faites sur les effets de la Naltrexone et du fragment 1-27 de la Bêta-endorphine permet donc de préconiser l'utilisation des antagonistes de la morphine dans le traîtement des états immuno-déficitaires acquis, et en particulier de l'infection par le VIH.

La présente invention a donc pour objet l'utilisation des antagonistes de la morphine, notamment vis-à-vis du récepteur mu, comme ingrédients actifs dans la préparation d'un médicament immunomodulateur et/ou antiviral destiné au traitement des états immunodéficitaires acquis, et en particulier de l'infection par le VIH.

Parmi les antagonistes de la morphine utilisables selon l'invention, on citera en particulier la Naltrexone, et ses analogues structuraux tels que la bêta-Chlornaltrexamine, le Nalméphène, la Naloxazone, la Naltrexonazine, etc ... ainsi que leurs dérivés tels que leurs sels pharmaceutiquement acceptables, notamment leurs chlorhydrates, citrates, sulfates, etc ... ainsi que les dérivés N-quaternaires correspondants. Les dérivés N-quaternaires sont par exemple quaternisés par l'alkylation de ces composés avec un groupement alkyle inférieur ayant par exemple 1 à 4 atomes de carbone. Ces dérivés sont préparés selon les méthodes connues. La Naltrexone est actuellement préférée, car sa sureté d'emploi a déjà été démontrée dans le traîtement de soutien, après la crise de sevrage, des toxicomanes opiacés-dépendants, comme préconisé dans le brevet U.S. N° 3.332 950. L'utilisation au long cours de la Naltrexone est en effet dénuée d'effets indésirables majeurs et est caractérisée par l'absence de phénomènes d'accoutumance ainsi que par une bonne tolérance aux doses préconisées. En outre, la Naltrexone présente l'avantage de pouvoir être administrée par voie orale et franchit sans difficulté la barrière hématoencéphalique.

Dans certains cas, on peut préférer utiliser des médicaments à action strictement périphérique ne franchissant pas la barrière hémato-encéphalique, comme c'est le cas avec les dérivés N-quaternisés de la Naltrexone et de ses analogues, par exemple un halogénure, tel que le bromure de N-méthyl Naltrexone.

On rappelle que la Naltrexone est la 17-(cyclopropylméthyl)-4,5-époxy-3,14-dihydroxymorphinan-6-one.

Parmi les antagonistes de la morphine utilisables selon l'invention, on citera également le fragment 1-27 de la bêta-endorphine humaine, et, plus généralement les peptides comportant 6 à 30 motifs d'acides aminés et dont l'extrémité C-terminale comporte la séquence minimale de formule I ou II :

..-Ile-Ile-Lys-Asn-Ala-His-CO₂H (I)

..-Ile-Ile-Lys-Asn-Ala-Tyr-CO₂H (II)

qui est responsable de la fixation sur les récepteurs mu ; ainsi que les dérivés et analogues de ces peptides comportant des modifications destinées par exemple à éviter leur dégradation rapide par les peptidases ou à favoriser leur passage à travers la barrière hématoencéphalique.

La préparation de ces peptides est effectuée selon les méthodes connues, soit par dégradation des peptides naturels, soit par synthèse ou hémi-synthèse. De même, la préparation de dérivés de ces peptides, non facilement dégradables, comportant par exemple des insertions d'acides aminés N-méthylés, ou de configuration D, ou d'autres dérivés d'acides aminés non naturels, est mise en oeuvre selon des procédés connus en soi ; voir par exemple B. RAJASHEKHAR et al ; J. BIOL. CHEM. ;261 (29), 13617 (1986).

L'activité antagoniste de la morphine, pour la bêta-endorphine 1-27, a été décrite par HAMMONDS et al., article cité.

Parmi les antagonistes de la morphine on peut citer également l'alpha-mélanotropine (dite aussi alpha-MSH) désacétylée ; ou encore certains fragments de l'ACTH, dépourvus d'activité corticotrope, tels que les fragments 1-16, 5-16, 5-14 et la D-Phé-ACTH 4-10 (Phé représente la phénylalanine) ; voir GISPEN, Eur. J. Pharmac., 39,393-397 (1976).

La préparation des médicaments contenant l'antagoniste de la morphine est effectuée de façon connue en soi. Par exemple, on mélange le principe actif avec un diluant, véhicule, adjuvant ou excipient pharmaceutique approprié permettant l'administration du médicament par voie orale, rectale, vaginale ou parentérale (y compris par voie sous-cutanée, intraveineuse, endonasale ou intrathécale).

Le principe actif est généralement présent dans ces médicaments à raison de 0,05 à 50 % en poids.

La voie orale est en règle générale réservée aux principes actifs non peptidiques, et la voie endonasale aux principes actifs peptidiques. La voie intrathécale est possible pour les deux types de principes actifs, avec si nécessaire un dispositif ad hoc de perfusion continue ou discontinue à débit constant ou variable.

Les formes pharmaceutiques utilisables sont notamment les suivantes : comprimés nus ou enrobés, dragées, capsules, tablettes sécables ou non, comprimés à enrobage protecteur, comprimés à libération progressive, microcapsules à enrobage entérique, capsules molles, gélules, pilules, cachets, solutés injectables, solutés pour pulvérisation nasale, suppositoires, et gélules à usage gynécologique, ainsi que solutions, sirops ou suspensions buvables, ou encore préparations lyophilisées pour solutions injectables à reconstituer au moment de l'emploi.

La posologie des médicaments à effet immunomodulateur et/ou anti-viral de l'invention varie en fonction de la voie d'administration, de la fréquence du traitement, de la phase de la maladie, de l'âge et du poids du sujet traité, et aussi de la réaction individuelle du malade.

En général, on administre de 0,02 à 2 mg de principe actif par kg de poids corporel par jour.

Pour la Naltrexone et ses dérivés et analogues structuraux, la dose moyenne quotidienne, par voie orale, est généralement de 30 à 70 mg, en particulier 50 mg, de principe actif par jour pour un adulte, en une ou plusieurs prises. Les doses sont de préférence augmentées graduellement. Par exemple, pour la voie orale, la dose administrée est de 5 à 10 mg le premier jour, puis de 20 mg le jour suivant, de 25 à 30 mg le troisième jour, etc ..., pour arriver progressivement à la posologie quotidienne choisie. En cas de toxicomanie associée aux opiacés exogènes, ces médicaments ne seront administrés, de préférence, qu'après un sevrage de 7 à 10 jours.

Dans les cas de malades ne réagissant que faiblement à ces médicaments, il est possible d'administrer des doses plus fortes pendant un temps limité, par exemple des doses pouvant atteindre 150 à 500 mg de principe actif par jour pour un adulte.

Dans le cas de l'utilisation selon l'invention de dérivés peptidiques comme ingrédients actifs, on administre généralement de 0,02 à 0,1 mg par kg et par jour chez l'adulte, par voie parentérale, en particulier par voie sous-cutanée, endonasale ou intrathécale.

Pour l'administration par voie intrathécale, des doses réduites sont utilisées, par exemple 0,05 à 0,1 mg/kg de principe actif, peptidique ou non peptidique, par jour.

Les médicaments décrits ci-dessus peuvent être administrés par exemple chez les sujets souffrant de déficits immunitaires acquis, notamment dans le cas des infections par le HIV, des états d'immunodépression, y compris les états d'immunodépression liés à la gravidité, ou encore des affections à composante auto-immune. L'administration chez la femme enceinte n'est pas contre-indiquée, les principes actifs utilisés n'ayant pas d'effet tératogène.

Les médicaments selon l'invention peuvent être utilisés dans le traîtement des infections par des virus opportunistes (par exemple virus herpès, virus d'Epstein-Barr, cytomégalovirus, papillomavirus, etc...)

Une période initiale de 1 à 3 mois de traitement est généralement conseillée. Cette période peut éventuellement être prolongée en fonction de l'appréciation clinique. Pendant le traitement avec la Naltrexone ou ses analogues et dérivés, il est recommandé de surveiller périodiquement les transaminases sériques, afin de vérifier la tolérance hépatique.

Ces médicaments à effet immunomodulateur et/ou anti-viral peuvent être utilisés, selon l'invention, en association avec d'autres chimiothérapies antivirales, ou antimitotiques, le cas échéant.

Ils peuvent aussi être associés à une substance potentialisant l'action des opioïdes endogènes à effet immunostimulant tels que par exemple la Met-enképhaline (ligand des récepteurs delta). Parmi ces substances potentialisatrices, on citera notamment le zinc (sous forme d'acétate, d'orotate ou de tout autre sel pharmaceutiquement acceptable, par exemple à la dose de 20 à 50 mg par jour chez l'adulte, par voie orale) ou encore les inhibiteurs des enképhalinases. Ils peuvent également être associés à des inhibiteurs d'aminopeptidases, à des inhibiteurs de la libération de la bêta-endorphine tels que la dopamine, la L-dopa, la bromocriptine (agoniste dopaminergique), ou encore les agonistes des récepteurs périphériques et centraux des benzodiazépines, en particulier les dérivés triazolés.

Les compositions pour la voie orale contenant du zinc contiennent aussi, de préférence du lactose en quantité suffisante, par exemple 75 à 90 mg par dose unitaire, pour favoriser l'absorption digestive du zinc.

On peut en outre associer aux médicaments à effet immunomodulateur et antiviral selon l'invention certains nutriments favorisant la réponse immunitaire, tels que le sélénium, les vitamines E, C, les vitamines du groupe B, en particulier B6 et B12, les folates, ou encore certains acides gras polyinsaturés.

L'invention permet de préparer des médicaments immunomodulateurs et/ou antiviraux contenant des peptides choisis parmi ceux qui comprennent de 6 à 30 acides aminés et dont l'extrémité. C-terminale comporte la séquence minimale de formule I ou II, donnee ci-dessus,ou encore l'alpha-MSH désacétylée et les fragments de l'ACTH mentionnés ci-dessus ; ainsi que les dérivés ou analogues de ces peptides comportant des acides aminés N-méthylés ou de configuration D. Ces médicaments contiennent l'ingrédient actif en association avec un véhicule, excipient ou diluant pharmaceutique approprié, outre l'ingredient actif présent par exemple à raison de 0,05 à 50 % en poids.

On peut ainsi mettre en oeuvre une méthode de traitement des états immuno-déficitaires acquis, et en particulier de l'infection par le VIH, caractérisée par le fait que l'on administre au patient une quantité efficace d'au moins un médicament immunomodulateur et/ou antiviral tel que défini ci-dessus.

Les exemples suivants illustrent l' invention sans toutefois la limiter.

### Exemples de réalisation de compositions pharmaceutiques

Dans une préparation destinee a une administration orale, telle qu'un comprime, on combine le principe actif, tel que la Naltrexone, par exemple à la dose de 50 milligrammes par unité d'administration, avec le lactose et avec un véhicule inerte non toxique par voie orale, pharmaceutiquement acceptable, tel que l'amidon (qualité pharmaceutique).

On peut également incorporer des liants, lubrifiants, agents de désagrégation et colorants appropriés.

Des exemples de liants comprennent à titre non limitatif l'amidon, la gélatine, la cellulose, ou encore le lactose, et des cires.

Un exemple de lubrifiant à utiliser dans ces formes dosées peut être à titre non limitatif l'acide stéarique.

Les agents de désagrégation appropriés sont par exemple, l'amidon ou encore la cellulose.

Si on le désire, on peut encore incorporer un colorant classique pharmaceutiquement acceptable, par exemple l'azorubine, le jaune de quinoléine ou l'indigotine.

Enfin, un enrobage ou un vernissage peuvent être pratiqués.

Il peut s'agir de comprimés simples contenant le seul principe actif ci-dessus mentionné, en association ou en mélange avec un véhicule ou un excipient inerte non toxique pharmaceutiquement acceptable ; de comprimés dits biphasiques dans lesquels un enrobage approprié tel qu'un polymère naturel comme la gélatine, ou encore un ester gras de glycérol comme le précirol sépare de façon stable, après vernissage de ce premier noyau, le principe actif de l'acétate de zinc et du lactose ; enfin de comprimés dits "tricouches" préparés suivant les techniques usuelles de l'industrie.

Pour l'administration par voie parentérale, on prépare des solutions aqueuses isotoniques, avec éventuellement un agent conservateur. On peut également préparer, à partir de solutions aqueuses, des compositions lyophilisées, contenant généralement un adjuvant de lyophilisation tel que le mannitol, le lactose ou analogue. Ces compositions lyophilisées sont utilisées pour reconstituer, au moment de l'emploi, des solutions injectables ou nébulisables. Les préparations injectables sous forme de solutions ou de poudres lyophilisées peuvent contenir un sel de zinc.

### EXEMPLE 1

On prépare suivant la technique usuelle des comprimés ayant la composition suivante :

| | mg/comprimé |
|---|---|
| - Naltrexone (chlorhydrate) | 50 |
| - lactose | 85 |
| - amidon | 40 |
| - stéarate de sodium | 2 |
| - acide stéarique | 5 |

On administre quotidiennement 1 comprimé. On prépare de façon analogue des comprimés dosés à 10, 20, 30 ou 40 mg de principe actif.

### EXEMPLE 2

On prépare suivant la technique usuelle des comprimés dits biphasiques formés d'un premier noyau séparant le principe actif, de façon stable, de l'acétate de zinc et du lactose, grâce à un enrobage à sec par le précirol liant, suivi d'un vernissage approprié.

| | mg/comprimé |
|---|---|
| - Naltrexone (chlorhydrate) | 50 |
| - précirol | 4 |
| - acétate de zinc | 45 |
| - lactose | 85 |
| - amidon | 15 |
| - stéarate de magnésium | 5 |
| - acide stéarique | 2 |

On administre quotidiennement un comprimé chez l'adulte.

### EXEMPLE 3

On prépare suivant la technique habituelle des comprimés dits biphasiques formés d'un premier noyau séparant le principe actif, de façon stable, de l'acétate de zinc et du lactose, grâce à un enrobage à sec par la gélatine suivi d'un vernissage approprié :

| | mg/comprimé |
|---|---|
| - Naltrexone (chlorhydrate) | 50 |
| - gélatine | 5 |
| - acétate de zinc | 45 |
| - lactose | 85 |
| - amidon | 15 |
| - stéarate de magnésium | 5 |
| - acide stéarique | 2 |

On administre quotidiennement un comprimé chez l'adulte.

### EXEMPLE 4 : Solution Injectable

La composition de cette solution, rapportée à 1 cm3, est la suivante :

| | |
|---|---|
| - Naltrexone (chlorhydrate) | 0,5 mg |
| - Chlorure de sodium | 9 mg |
| - Parabenzoate de méthyle | 1,5 mg |
| - Parabenzoate de propyle | 0,1 mg |
| - Eau distillée apyrogène, qsp | 1 cm³ |

On répartit cette solution dans des ampoules de 2 cm³ ou de 5 cm³

### EXEMPLE 5 :Composition lyophilisée

Cette composition contient :

| | |
|---|---|
| - bêta-endorphine 1-27 | 0,3 mg |
| - lactose | 30 mg |

Par dissolution de cette composition avec 3 cm3 d'une solution aqueuse stérile apyrogène à 0,9 % de NaCl, on obtient une solution injectable.

### EXEMPLE 6 :Soluté pour pulvérisations nasales

Composition :

| | |
|---|---|
| - Bêta-endorphine 1-27 | 30 mg |
| - Excipient (phosphate disodique anhydre, chlorure de sodium, sorbitol, glycérol, agents conservateurs, eau distillée) qsp | 10 ml |

On pulvérise environ 0,1 ml dans chaque narine 4 à 6 fois par jour.

## Revendications

1. Utilisation des antagonistes de la morphine ayant une affinité pour les récepteurs mu ou pour les récepteurs mu et kappa, comme ingrédients actifs dans la préparation d'un médicament immunomodulateur et/ou antiviral destiné au traitement des déficits acquis de l'immunité cellulaire.

2. Utilisation selon la revendication 1 dans la préparation d'un médicament destiné au traitement des infections par le VIH.

3. Utilisation selon l'une quelconque des revendications précédentes, caractérisée par le fait que ledit antagoniste est choisi parmi la Naltrexone, la bêta-chlornaltrexamine, le Nalméphène, la Naltrexonazine et la Naloxazone, ainsi que leurs sels pharmaceutiquement acceptables et les dérivés N-quaternaires correspondants.

4. Utilisation selon la revendication 3 caractérisée par le fait que les dérivés N-quaternaires sont quaternisés avec un groupement alkyle inférieur.

5. Utilisation selon l'une quelconque des revendications 1 et 2, caractérisée par le fait que ledit antagoniste est un peptide camportant 6 à 30 motifs d'acides aminés et dont l'extrémité C-terminale comporte la séquence minimale de formule I ou II:
- Ile-Ile-Lys-Asn-Ala-His-CO₂H (I)
- Ile-Ile-Lys-Asn-Ala-Tyr-CO₂H (II)
ainsi que les dérivés et analogues de ces peptides comportant des acides aminés N-méthylés ou de configuration D.

6. Utilisation selon la revendication 5, caractérisée par le fait que ledit peptide est le fragment 1-27 de la bêta-endorphine.

7. Utilisation selon l'une quelconque des revendications 1 et 2, caractérisée par le fait que ledit antagoniste est l'alpha-mélanotropine désacétylée.

8. Utilisation selon l'une quelconque des revendications 1 et 2, caractérisée par le fait que ledit antagoniste est choisi parmi les fragments 1-16, 5-16 et 5-14 de l'ACTH et la D-Phé-ACTH 4-10.

9. Utilisation selon l'une quelconque des revendications précédentes, caractérisée par le fait que ledit médicament est destiné à être administré en association avec un sel de zinc pharmaceutiquement acceptable.

## Claims

1. The use of morphine antagonists having an affinity for mu receptors or mu and kappa receptors as active ingredients in the preparation of an immunomodulator and/or antiviral drug intended for the treatment of acquired cellular immunity deficiencies.

2. A use according to claim 1 in the preparation of a drug intended for the treatment of HIV infections.

3. A use according to one or other of the preceding claims, characterized in that the said antagonist is selected from Naltrexone, beta-chloronaltrexamine, Nalmephene, Naltrexonazine and Naloxazone and their pharmaceutically-acceptable salts and the corresponding quaternary nitrogen derivatives.

4. A use according to claim 3, characterized in that the quaternary nitrogen derivatives are converted to quaternary nitrogen form with a lower alkyl group.

5. A use according to one or other of claims 1 and 2, characterised in that the said antagonist is a peptide comprising 6 to 30 amino acid structural units, the terminal carbon end of which comprises a minimum sequence corresponding to formula I or II:
- Ile-Ile-Lys-Asn-Ala-His-CO₂H (I)
- Ile-Ile-Lys-Asn-Ala-Tyr-CO₂H (II)
and the derivatives and analogues of these peptides comprising N-methyl amino acids or D configuration amino acids.

6. A use according to claim 5, characterised in that the said peptide is the 1-27 fragment of beta-endorphin.

7. A use according to one or other of claims 1 and 2, characterized in that the said antagonist is deacetylated alpha-melanotropin.

8. A use according to one or other of claims 1 and 2, characterised in that the said antagonist is selected from the 1-16, 5-16 and 5-14 fragments of ACTH and D-Phe-ACTH 4-10.

9. A use according to any one of the preceding claims, characterised in that the said drug is intended to be administered in association with a pharmaceutically acceptable zinc salt.

## Patentansprüche

1. Verwendung von Morphinantagonisten mit Affinität für µ-Rezeptoren oder für µ- und κ-Rezeptoren als Wirkstoffe bei der Herstellung eines immunomodulatorischen und/oder antiviralen Medikaments, das zur Behandlung von erworbenen zellulären Immunmangelzuständen bestimmt ist.

2. Verwendung nach Anspruch 1, bei der Herstellung eines Medikaments, das zur Behandlung von HIV-Infektionen bestimmt ist.

3. Verwendung nach einem der vorstehenden Ansprüche, dadurch gekennzeichnet, daß der Antagonist unter Naltrexon, ß-Chlornaltrexamin, Nalmephen, Naltrexonazin und Naloxazon sowie von pharmazeutisch verträglichen Salzen und entsprechenden N-quaternären Derivaten ausgewählt ist.

4. Verwendung nach Anspruch 3, dadurch gekennzeichnet, daß die N-quaternären Derivate mit einer niederen Alkylgruppe quaternisiert sind.

5. Verwendung nach einem der Ansprüche 1 und 2, dadurch gekennzeichnet, daß es sich beim Antagonisten um ein Peptid mit 6 bis 30 Aminosäureresten handelt, dessen C-terminaler Rest die Mindestsequenz der Formel I oder II aufweist:
- Ile-Ile-Lys-Asn-Ala-His-CO₂H (I)
- Ile-Ile-Lys-Asn-Ala-Tyr-CO₂H (II)
sowie von Derivaten und Analogen dieser Peptide, die N-methylierte Aminosäuren oder Aminosäure mit D-Konfiguration aufweisen.

6. Verwendung nach Anspruch 5, dadurch gekennzeichnet, daß es sich bei dem Peptid um das Fragment 1-27 von ß-Endorphin handelt.

7. Verwendung nach einem der Ansprüche 1 und 2, dadurch gekennzeichnet, daß es sich beim Antagonisten um desacetyliertes α-Melanotropin handelt.

8. Verwendung nach einem der Ansprüche 1 und 2, dadurch gekennzeichnet, daß der Antagonist unter den Fragmenten 1-16, 5-16 und 5-14 von ACTH und D-Phe-ACTH 4-10 ausgewählt ist.

9. Verwendung nach einem der vorstehenden Ansprüche, dadurch gekennzeichnet, daß das Medikament zur Verabreichung in Verbindung mit einem pharmazeutisch verträglichen Zinksalz vorgesehen ist.
